(19) Europäisches Patentamt
European Patent Office
Office européen des brevets

(11) **EP 4 629 250 A1**

(12) **EUROPEAN PATENT APPLICATION**
published in accordance with Art. 153(4) EPC

(43) Date of publication:
**08.10.2025 Bulletin 2025/41**

(21) Application number: **23897620.3**

(22) Date of filing: **21.11.2023**

(51) International Patent Classification (IPC):
***G16C 10/00*** (2019.01)

(52) Cooperative Patent Classification (CPC):
**G16C 10/00**

(86) International application number:
**PCT/JP2023/041819**

(87) International publication number:
**WO 2024/116972 (06.06.2024 Gazette 2024/23)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.11.2022 JP 2022190589**

(71) Applicant: **Panasonic Intellectual Property Management Co., Ltd.
Kadoma-shi, Osaka 571-0057 (JP)**

(72) Inventors:
• **MAEDA Suguru
Kadoma-shi, Osaka 571-0057 (JP)**
• **HIBINO Mitsuhiro
Kadoma-shi, Osaka 571-0057 (JP)**

(74) Representative: **Novagraaf International SA
Chemin de l'Echo 3
1213 Onex, Geneva (CH)**

(54) **METHOD FOR EVALUATING HOPPING CONDUCTION PROPERTIES OF MATERIAL**

(57) A disclosed method for evaluating hopping conduction physical properties includes: a first calculation step of calculating, by a density functional method or a molecular dynamics method, a state quantity of a state in which charge is localized on each of N atoms constituting a material M; a second calculation step of calculating, from the calculation result of the first calculation step, a probability Px where x = 1 to N that charge is localized on each of the N atoms; and a decision step of selecting atoms having a high probability Px, to decide a charge transfer pathway.

*FIG. 5*

Two potential curves are drawn separately, the intersection of which is determined as an activation energy (double-headed dotted arrow).

**Description**

[Cross Reference to Related Application]

The present disclosure claims priority with respect to the Japanese Patent Application No. 2022-190589 filed on November 29, 2022, and the entire content of the patent application is incorporated herein by reference into the present specification.

[Technical Field]

The present disclosure relates to a method for evaluating hopping conduction physical properties of a material.

[Background Art]

Hopping conduction is a conduction mechanism in which electric charge localized on a certain atom moves to a nearby atom. Regarding the evaluation of hopping conduction, the following reports are available, for example.
Non-Patent Literature 1 proposes a multiple scattering theory and a variable range hopping (VRH) model, based on a first-principle calculation method using a coherent potential approximation.
In Non-Patent Literature 2, the formation and migration of hole polarons in bulk rutile and anatase-type titania are modeled, and simulated using a density functional method. Here, the Marcus/Holstein theory of electron/polaron transfer is combined.
Non-Patent Literature 3 discusses the conduction mechanism of oxygen ions and electrons from atomistic and electronic level viewpoints, by applying a density functional method.
Non-Patent Literature 4, using the multiple scattering theory and the variable region hopping (VRH) model proposed by Mott based on a density functional method using a coherent potential approximation, evaluates low-temperature DC conductivity and its temperature dependency for n-doped wurtzite-type ZnO.
Non-Patent Literature 5 discusses the relationship between the energy level mobility and the conductivity related to hopping conduction, based on Mott's variable region hopping (VRH) model.

[Citation List]

[Patent Literature]

Non-Patent Literature 1: N. F. Mott, J. Non-Cryst. Solids, 1968, 1, 1-17.
Non-Patent Literature 2: N. Aaron Deskins et. Al., J. Phys. Chem. C, 2009, 113, 346-3 58.
Non-Patent Literature 3: M. Nakayama et. Al., Phys. Chem. Chem. Phys., 2012, 14, 6079-6084.
Non-Patent Literature 4: R. Plugaru et al., Results Phys., 2012, 2, 190-197.
Non-Patent Literature 5: G. Paasch et al., Synth Met., 2002, 132, 97-104.

[Summary of Invention]

[Technical Problem]

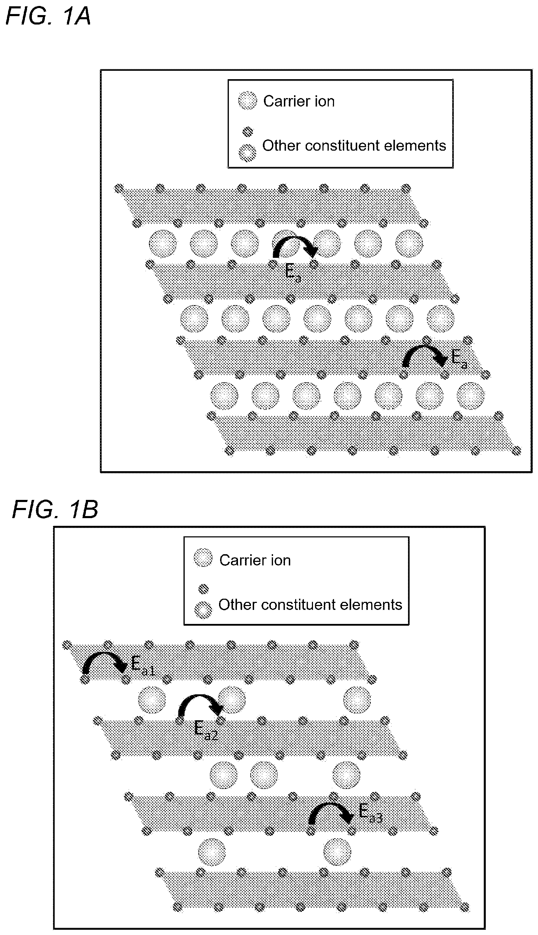

Predicting the conductivity due to hopping conduction gives useful information when selecting an electrode material for electrochemical devices, such as secondary batteries. When the physical properties of the electrode material are to be evaluated in detail, it is desired to evaluate the hopping conduction physical properties even for the electrode material in the middle of charging and discharging. The conventional method for evaluating hopping conduction, however, has been performed on the assumption that the activation energy is similar for all positions in the crystal structure.
For example, as shown in FIG. 1A, in the crystal structure of a positive electrode material in a fully discharged state, almost all carrier ion sites are occupied by carrier ions. In that case, the electronic state within the crystal can be seen as a repetition of a small unit like a primitive cell. It suffices therefore to calculate the activation energy at one location within the crystal, for calculating the conductivity, etc.
However, for example, in the case of a material to and from which carrier ions are repeatedly inserted and desorbed, carrier ions randomly enter and exit in association with charging and discharging. That is, the activation energy differs position by position due to the irregular arrangement of carrier ions. It is therefore fundamentally unreasonable to decide one value of the activation energy. FIG. 1B conceptually illustrates a crystal structure of a positive electrode material in the middle of charging and discharging. In the positive electrode material in the middle of charging and discharging, carrier ions randomly occupy the carrier ion sites.

[Solution to Problem]

One aspect of the present disclosure related to a method for evaluating hopping conduction physical properties, including: a first calculation step of calculating, by a density functional method or a molecular dynamics method, a state quantity of a state in which charge is localized on each of N atoms constituting a material M; a second calculation step of calculating, from the calculation result of the first calculation step, a probability $P_x$ where $x = 1$ to N that charge is localized on each of the N atoms; and a decision step of selecting atoms having a high probability $P_x$, to decide a charge transfer pathway.

[Advantageous Effects of Invention]

According to the present disclosure, it is possible to evaluate hopping conduction physical properties of a material in any state.

While the novel features of the invention are set forth particularly in the appended claims, the invention, both as to organization and content, will be better understood and appreciated, along with other objects and features thereof, from the following detailed description taken in conjunction with the drawings.

[Brief Description of Drawings]

[FIG. 1A] A conceptual diagram of the crystal structure of a positive electrode material having a layered structure in a fully discharged state, in which all the carrier ion sites are occupied by carrier ions.
[FIG. 1B] A conceptual diagram of the crystal structure of a positive electrode material having a layered structure in the middle of charging and discharging, in which the carrier ion sites are randomly occupied by carrier ions.
[FIG. 2] A conceptual diagram showing an example of a probability $P_x$ that charge is localized, determined for each of N atoms.
[FIG. 3] A conceptual diagram in which (a) shows a charge hopping pathway that traverses a calculation cell and (b) shows infinite hopping through the material M.
[FIG. 4] A conceptual diagram showing n intermediate states between a state in which charge is localized on an atom x and a state in which charge is localized on an atom x'.
[FIG. 5] A conceptual diagram showing two potential curves drawn in a coordinate system having an energy axis and a reaction axis.
[FIG. 6] A diagram showing changes in activation energy along an example of a hopping conduction path.

[Description of Embodiments]

Embodiments of the present disclosure will be described below by way of examples, but the present disclosure is not limited to the examples described below. In the following description, specific numerical values and materials are exemplified in some cases, but other numerical values and other materials may be adopted as long as the effects of the present disclosure can be obtained. For the components other than those characteristic of the present disclosure, any known components for electrochemical devices, such as primary batteries and secondary batteries, may be adopted. In the present specification, when referring to "a range of a numerical value A to a numerical value B," the range includes the numerical value A and the numerical value B, and can be rephrased as "a numerical value A or more and a numerical value B or less." For example, "A to B mol%" is equivalent to "A mol% or more and B mol% or less." In the following description, when the lower and upper limits of numerical values related to specific physical properties, conditions, etc. are mentioned as examples, any one of the mentioned lower limits and any one of the mentioned upper limits can be combined in any combination as long as the lower limit is not equal to or more than the upper limit. When a plurality of materials are mentioned as examples, one kind of them may be selected and used singly, or two or more kinds of them may be used in combination.

A prediction target of the conductivity due to hopping conduction according to the present disclosure is assumed to be an electrode material for electrochemical devices, such as primary batteries and secondary batteries, but is not limited thereto. One example of the electrode material is a material capable of a Faradaic reaction through which lithium ions are absorbed and released. Such a material include a metal, a metal compound, a carbon material, and the like. The metal can be a metal that can form an alloy with lithium. The metal compound can be an oxide, a sulfide, a halide, a nitride, a polyanion salt, and the like. A typical example of the oxide is a lithium-containing metal oxide, and is assumed to be an oxide having a crystal structure of spinel-type, perovskite-type, rock-salt type, or the like. A typical example of the polyanion salt is assumed to be a salt having a crystal structure of olivine type.

A method for evaluating hopping conduction physical properties according to the present disclosure is applicable, for example, as a method for evaluating the conductivity due to hopping conduction. The evaluation method includes a first calculation step, a second calculation step, and a decision step as below.

<First calculation step>

The first calculation step is a step of calculating, by a density functional method or a molecular dynamics method, a state quantity of a state in which charge is localized on each of N atoms constituting a material M. An example of the state quantity includes, for example, an energy state. The energy states of the N atoms and electrons in each atom can be calculated based on their atomic arrangement. The calculation is performed on the assumption that, in the material M, N atoms have an intrinsic atomic arrangement.

Here, when the atomic arrangement is not known, as a preliminary step before performing the first calculation step, first, the atomic arrangement of the material M is decided. In the following, description will be given with a lithium-containing composite oxide having a layered rock-salt type crystal structure taken as an example.

Specifically, first, a structure file of the material M is created. The structure file may be created by any method, which may be downloaded from the crystal structure database, or created using a crystal structure drawing software, based on the information described in papers.

In the case of evaluating the hopping conduction physical properties of a material in any state in the middle of charging and discharging, the structure file is rewritten to create a random structure file in which carrier ions such as lithium ions are removed by the number as desired.

Next, based on the structure file, the state quantity of a state in which charge is localized on each of the N atoms constituting the material M is calculated. Specifically, a calculation using a molecular dynamics method or a calculation using a density functional method (hereinafter, sometimes referred to as a "DFT calculation") is performed for all the atoms.

For example, in the DFT calculation, a calculation is performed, with the crystal structure (initial atomic position), the potential of each atom, the calculation condition, and the like used as the input information, and the stable structure (stable atomic position), the electronic structure, the energy, the charge density, the electron spin, and the like used as the output information. For the DFT calculation, a publicly known first-principle calculation software may be used.

The first-principle calculation software executes calculation, for example, based on the input information, such as the matrix indicating the position of a calculation cell in the material M, the number of atoms, and the matrix indicating the position of each atom. Here, the "calculation cell" refers to a structural region where the material M is modeled. It suffices to set a calculation cell having a sufficient size for evaluation of hopping conduction, without limitation to the size of the calculation cell.

An electron correlation energy U [eV] is assigned to each atom contained in the calculation cell. Specifically, with respect to one structure determined, for a certain atom, an electron correlation energy U [eV] is set, on the basis of the calculation condition file (in other words, the input information in the DFT calculation).

The electron correlation energy U is an energy that destabilizes the electrons in a specific orbital, and is a common theory used for transition metals and the like. In the present disclosure, by using the electron correlation energy U as an "energy to expel electrons from a certain orbit", a calculation is performed for an energy $E_x$ (x = 1 to N) of the state in which electrons have been expelled from a specific atom x to cause localization of positive charge and in which charge is localized on that atom x.

In short, the information on an arbitrary atom and the electron correlation energy U [eV] related to that atom is inputted in a first principle calculation software, and a calculation is performed. Thus, the stable structure (stable atomic position), the electronic structure, the energy, the charge density, the electron spin, and the like when charge is localized on that atom can be determined.

<Second calculation step>

Next, in the second calculation step, from the calculation result in the first calculation step, a probability $P_x$ (x = 1 to N) that charge is localized on each of the N atoms is calculated. The method for determining the probability $P_x$ is not particularly limited. In the case where the calculation result when charge is localized on each atom is obtained in the first calculation step, using that result, the probability $P_x$ that charge is localization can be determined by any method. Specifically, supposing that charge is localized on an atom (1) of N = 1, a calculation is performed with an electron correlation energy U assigned to the atom (1), and then, the energy for the total atom at that time can be obtained. That is, one probability $P_1$ is obtained when charge is localized on the atom (1). By repeating this calculation N times to the atom (N), N calculation results in total can be obtained.

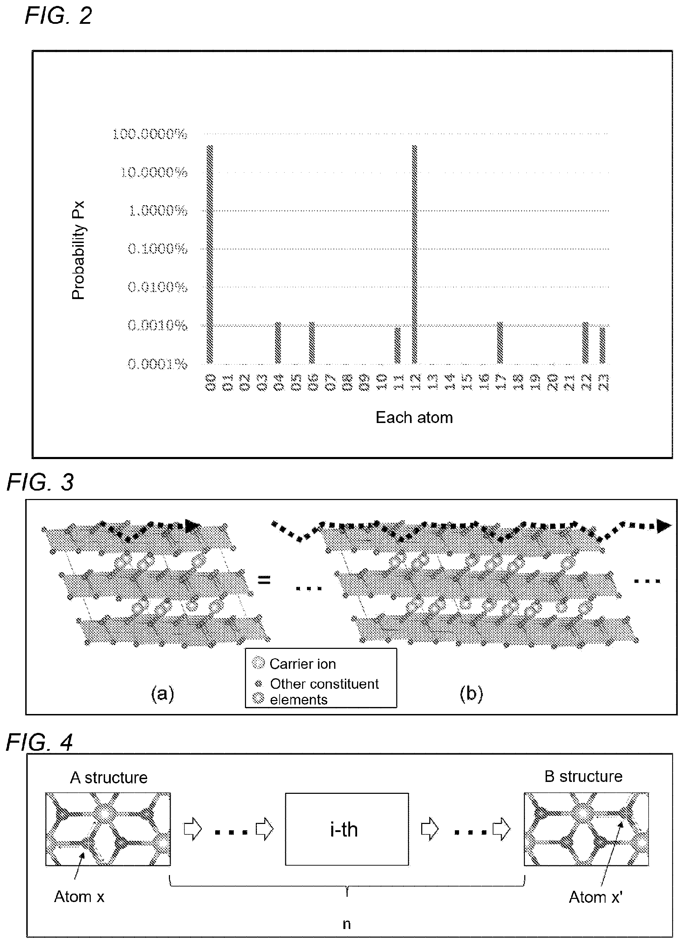

Although any method can be used for determining the probability Px, the probability Px is desirably determined using, as a parameter, for example, at least one selected from the group consisting of the charge density, the energy, the electron spin, the local distortion, and the point defect. The probability Px can be converted, for example, into a numerical value for each atom as shown in FIG. 2. In the illustrated example, a total of 24 (x = 1 to 24) numerical values for atoms 00 to 23 are determined.

<Decision process>

Next, atoms having a high probability Px are selected, to decide a charge transfer pathway. Specifically, as shown in FIG. 3, the charge transfer pathway that traverses a calculation cell may be decided by connecting atoms having a high probability Px of the charge localization. It can be seen that deciding a charge hopping pathway (a) that traverses a calculation cell having a sufficient size is equivalent to calculating a pathway (b) of infinite hopping through the material M.
Specifically, first, it is to be judged whether or not the atoms having the first highest and the second highest probabilities Px are connected so as to traverse the calculation cell. Here, "connected" means that two atoms are located next to each other or two atoms apart, or within an effective Bohr radius ($a_B$) as described later. If there is no traversing pathway, it is to be judged how many of the atoms having the first highest, the second highest, and the third highest probabilities Px are connected so as to traverse the calculation cell. If there is still no traversing pathway, a similar processing is repeated, to judge whether or not the atoms having the first highest, the second highest, the third highest, the fourth highest ... probabilities Px are connected so as to traverse the calculation cell. In this way, when a bunch of atoms linked one after another that traverse the calculation cell is found for the first time, the bunch of atoms linked one after another is regarded as the hopping pathway.

<Third calculation step>

Next, an activation energy required for the charge transfer in the calculated charge transfer pathway is determined. An example of a method for determining the activation energy will be described below.
First, assuming that charge is localized on an arbitrary atom x included in the transfer pathway, the initial positions of all the atoms at that time have already been acquired as a stable structure (stable atomic position) in the first calculation step, which can be expressed as follows, for example.

<When charge is localized on atom x>

atom 1: (X coordinate: X1x Y coordinate: Y1x Z coordinate: Z1x)
atom 2: (X2x Y2x Z2 x)
..., and
atom N: (XNx YNx ZNx)

It is possible to determine an activation energy by determining an energy in the crystal structure in the middle of hopping from the state in which charge is localized on an atom x (x = 1 to N) to the state in which charge is localized on an atom x' (x' = 1 to N, and x' ≠ x). Since the energy during hopping cannot be determined continuously, here, a DFT calculation is performed by dividing into n intermediate (transitional) states as shown in FIG. 4. The respective atomic positions in n intermediate states between the state in which charge is localized on an atom x and the state in which charge is localized on an atom x' can be expressed as follows.

<Atomic position in i-th state in n intermediate states>

The crystal structure in the state in which charge is localized on an atom x is referred to as an A structure, and the crystal structure in the state in which charge is localized on an atom x' is referred to as a B structure. At this time, the crystal structure can be considered to change from the A structure to the B structure as a result of charge hopping. In the A structure and the B structure, when arbitrary atoms are connected by a straight line, the atomic position in the crystal structure in the intermediate state will be on the straight line.
Specifically, given that the respective atomic positions in the A structure in which charge is localized on an atom x are expressed as

atom 1: (X1x Y1x Z1x)
atom 2: (X2x Y2x Z2x)

..., and
atom N: (XNx YNx ZNx), and
the positions in the B structure in which charge is localized on an atom x' are expressed as
atom 1: (X1x' Y1x' Z1x')
atom 2: (X2x' Y2x' Z2x')
 ..., and
atom N: (XNx' YNx' ZNx'),
the positions of the respective atoms in the i-th state can be expressed as atom1: (X-coordinate: X1x(1-i/n) + X1x'(i/n)
Y-coordinate: Y1x(1-i/n) + Y1x'(i/n)
Z-coordinate: Z1x(1-i/n) + Z1x'(i/n))
atom 2: (X2x(1-i/n) + X2x'(i/n)

$$Y2x(1-i/n) + Y2x'(i/n)$$

$$Z2x(1-i/n) + Z2x'(i/n))$$

..., and
atom N: (XNx(1-i/n) + XNx'(i/n)

$$YNx(1-i/n) + YNx'(i/n)$$

$$ZNx(1-i/n) + ZNx'(i/n)).$$

Next, the atom x and the atom x' in the (n) states between the state in which charge is localized on the atom x and the state in which charge is localized on the atom x' are respectively multiplied by an electron correlation energy U, to perform a DFT calculation. At this time, unlike the ordinary DFT calculation, without moving the positions of the respective atoms, the electronic state at the atomic position in the i-th state expressed as above is calculated, and then, an energy value can be determined.

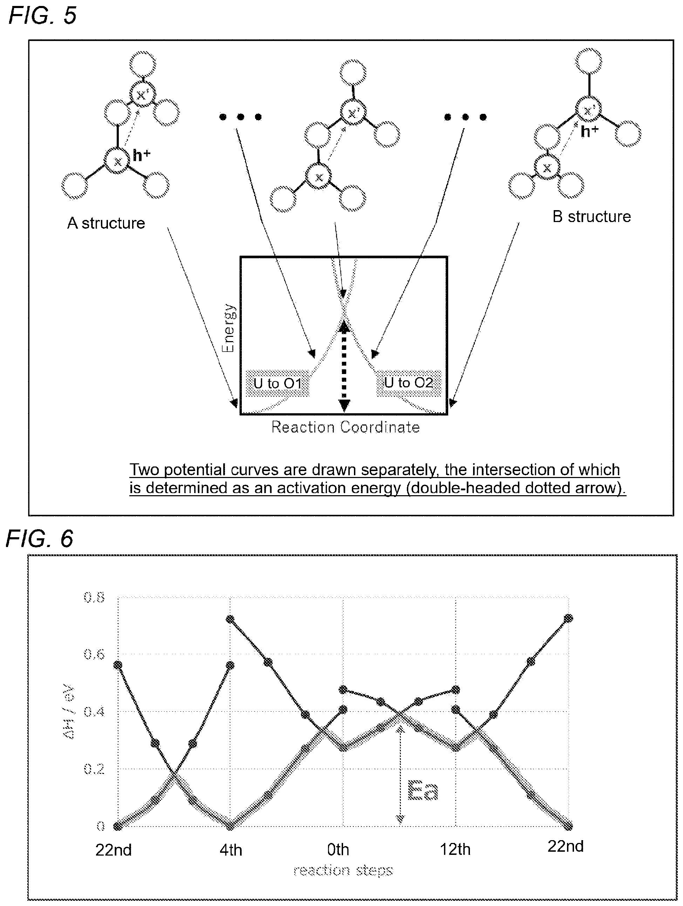

When (n) plots are drawn in a coordinate system with the reaction axis (the number of n states) as the horizontal axis and the obtained energy value as the vertical axis, and the resulting plots are connected, two potential curves as shown in FIG. 5 are obtained. In general, the DFT calculation on the atom x results in an upward-sloping curve, and the DFT calculation on the atom x' results in a downward-sloping curve. The intersection of the obtained two curves corresponds to an activation energy of the structural change from the A structure to the B structure.

The crystal structure changes as a result of hopping of charge from the atom x to the atom x', which is referred as having changed from the A structure to the B structure. When the charge hops along the "pathway traversing the calculation cell" decided in the above "decision step of selecting atoms having a high probability Px to decide the charge transfer pathway", the change is not limited to a change between two structures, and can be considered to sequentially change from the A structure, to the B structure, to the C structure, to .... In that case, the activation energy is determined for each of the structural changes from the A structure to the B structure, from the B structure to the C structure, from the C structure to the .... The A structure, B structure, C structure, ... are each a stable structure obtained in the first calculation step, which is, as described above, a structure obtained by performing a calculation, with an electron correlation energy U assigned to a predetermined atom. Assuming that the lowest energy state in all the structures is 0, the highest energy among the activation energies in the structural changes from the A structure to the B structure, from the B structure to the C structure, from the C structure to ... can be regarded as an activation energy $E_a$ in the hopping conduction in the material M. FIG. 6 shows changes in activation energy along an example of the hopping conduction pathway. The largest energy value when an energy curve is drawn continuously can be regarded as the activation energy $E_a$. In the case of FIG. 6, the hopping pathway that goes infinitely is a pathway of $22 \rightarrow 4 \rightarrow 0 \rightarrow 12 \rightarrow 22 \rightarrow 4 \rightarrow 0 \rightarrow 12 \rightarrow 22 \rightarrow ...$ that traverses the calculation cell and returns to the same position in the repeating unit. For example, there is a case where 22 (1st highest) $\rightarrow$ 4 (4th highest) $\rightarrow$ 0 (3rd highest) $\rightarrow$ 12 (2nd highest) $\rightarrow$ 22 (1st highest), and there can be a case where 22 (3rd highest) $\rightarrow$ 4 (6th highest) $\rightarrow$ 0 (2nd highest) $\rightarrow$ 12 (4th highest) $\rightarrow$ 22 (3rd highest).

<Fourth calculation step>

Subsequently, the conductivity may be calculated using the activation energy $E_a$. A conductivity $\sigma$ may be derived from an equation (1) and equations (1) to (4) modified from the equations derived by Mott.

[Math. 1]

$$\sigma = e^2 D N_F \quad \cdots (1)$$

$$D = R^2 v_{ph} \, exp\left(-2\alpha R - \frac{E_a}{k_B T}\right) \quad \cdots (2)$$

$$R = \left(\frac{9}{8\pi}\frac{1}{\alpha N_F k_B T}\right)^{1/4} \quad \cdots (3)$$

$$\frac{1}{\alpha} = a_B = \frac{4\pi\varepsilon\hbar^2}{m_e^* e^2} \quad \cdots (4)$$

In the equation, $E_a$ is an activation energy, e is a charge, D is a diffusion coefficient, $N_F$ is a density of state (density of electronic state) $\times$ Fermi-Dirac distribution, R is a hopping distance, $v_{ph}$ is a phonon frequency, $\alpha$ is a localization length (degree of broadening of the wave function of the carrier at the localized level), T is a temperature, $k_B$ is the Boltzmann constant, $a_B$ is an effective Bohr radius, $\varepsilon$ is a dielectric constant, and $\hbar$.
is the Dirac constant. Also, $m_e^*$ is an electron effective mass, and can be calculated using the band-dispersion curvature:

[Math. 2]

$$\frac{\partial^2 E_k}{\partial k^2}$$

as in an equation (5).
[Math. 3]

$$\frac{1}{m_e^*} = \frac{1}{\hbar^2}\frac{\partial^2 E_k}{\partial k^2} \quad \cdots (5)$$

The charge e is a quantity of the charge to hop. The "density of electronic state" in the density of electronic state $\times$ Fermi-Dirac distribution $N_F$ can be obtained by a DFT calculation. The Fermi-Dirac distribution is

$$f(E) = (exp((E\text{-}EF) / kBT) + 1)^{-1},$$

where E is an energy, and EF is a Fermi-level, both of which can be obtained by a DFT calculation. The hopping distance R can be determined from the positions of atoms obtained in the first calculation step. That is, the distance from an atom on which charge is localized before hopping to an atom on which charge is localized after hopping is determined. The phonon frequency $v_{ph}$ and the dielectric constant $\varepsilon$ may be experimental values or may be values obtained by separately performing a DFT calculation or a molecular dynamics method. The electron effective mass $m_e^*$ is obtained using the aforementioned band-dispersion curvature, and the band dispersion can be obtained by a DFT calculation.

<<Supplementary notes>>

The above description of embodiments discloses the following techniques.

(Technique 1)

A method for evaluating hopping conduction physical properties, comprising:

a first calculation step of calculating, by a density functional method or a molecular dynamics method, a state quantity of a state in which charge is localized on each of N atoms constituting a material M;
a second calculation step of calculating, from the calculation result of the first calculation step, a probability Px where x = 1 to N that charge is localized on each of the N atoms; and

a decision step of selecting atoms having a high probability Px, to decide a charge transfer pathway.

(Technique 2)

The method for evaluating hopping conduction physical properties according to technique 1, further comprising

an arrangement decision step of deciding an atomic arrangement of the N atoms, wherein
the first calculation step calculates the state quantity, based on the atomic arrangement.

(Technique 3)

The method for evaluating hopping conduction physical properties according to technique 1 or 2, further comprising a third calculation step for determining an activation energy required for charge transfer in the charge transfer pathway.

(Technique 4)

The method of evaluating hopping conduction physical properties according to any one of techniques 1 to 3, further comprising a fourth calculating step of calculating a conductivity using the activation energy.

(Technique 5)

The method of evaluating hopping conduction physical properties according to any one of techniques 1 to 4, wherein in the second calculation step, the probability Px is calculated using, as a parameter, at least one selected from the group consisting of a charge density, an energy, an electron spin, a local distortion, and a point defect of the N atoms.

Although the present invention has been described in terms of the presently preferred embodiments, it is to be understood that such disclosure is not to be interpreted as limiting. Various alterations and modifications will no doubt become apparent to those skilled in the art to which the present invention pertains, after having read the above disclosure. Accordingly, it is intended that the appended claims be interpreted as covering all alterations and modifications as fall within the true spirit and scope of the invention.

[Industrial Applicability]

The method for evaluating hopping conductivity physical properties according to the present disclosure is applicable, for example, in the development, selection, and the like of an electrode material for electrochemical devices, such as secondary batteries, and provides useful information regarding the conductivity of the electrode material.

**Claims**

1. A method for evaluating hopping conduction physical properties, comprising:

   a first calculation step of calculating, by a density functional method or a molecular dynamics method, a state quantity of a state in which charge is localized on each of N atoms constituting a material M;
   a second calculation step of calculating, from the calculation result of the first calculation step, a probability Px where x = 1 to N that charge is localized on each of the N atoms; and
   a decision step of selecting atoms having a high probability Px, to decide a charge transfer pathway.

2. The method for evaluating hopping conduction physical properties according to claim 1, further comprising

   an arrangement decision step of deciding an atomic arrangement of the N atoms, wherein
   the first calculation step calculates the state quantity, based on the atomic arrangement.

3. The method for evaluating hopping conduction physical properties according to claim 1, further comprising a third calculation step for determining an activation energy required for charge transfer in the charge transfer pathway.

4. The method of evaluating hopping conduction physical properties according to claim 3, further comprising a fourth calculating step of calculating a conductivity using the activation energy.

5. The method of evaluating hopping conduction physical properties according to claim 1, wherein in the second calculation step, the probability Px is calculated using, as a parameter, at least one selected from the group consisting of a charge density, an energy, an electron spin, a local distortion, and a point defect of the N atoms.

## FIG. 1A

## FIG. 1B

FIG. 2

FIG. 3

Carrier ion
Other constituent elements

(a)          (b)

FIG. 4

A structure

Atom x

i-th

n

B structure

Atom x'

*FIG. 5*

A structure

B structure

Energy

U to O1        U to O2

Reaction Coordinate

Two potential curves are drawn separately, the intersection of which is determined as an activation energy (double-headed dotted arrow).

*FIG. 6*

22nd        4th        0th        12th        22nd

reaction steps

## INTERNATIONAL SEARCH REPORT

| International application No. |
|---|
| **PCT/JP2023/041819** |

**A. CLASSIFICATION OF SUBJECT MATTER**

*G16C 10/00*(2019.01)i

FI: G16C10/00

According to International Patent Classification (IPC) or to both national classification and IPC

**B. FIELDS SEARCHED**

Minimum documentation searched (classification system followed by classification symbols)

G16C10/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | KICK, Matthias et al., Mobile Small Polarons Qualitatively Explain Conductivity in Lithium Titanium Oxide Battery Electrodes, The Journal of Physial Chemistry Letters, March 2020, vol. 11, pp. 2535-2540<br>entire text, all drawings | 1-5 |
| A | WU, Hao et al., Low-bias photoelectrochemical water splitting via mediating trap states and small polaron hopping, Nature Communications, October 2022, vol. 13, no. 6231, pp. 1-12<br>entire text, all drawings | 1-5 |

☐ Further documents are listed in the continuation of Box C.    ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **15 January 2024** | **23 January 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022190589 A

**Non-patent literature cited in the description**

- **N. F. MOTT**. *J. Non-Cryst. Solids*, 1968, vol. 1, 1-17
- **N. AARON DESKINS**. *J. Phys. Chem. C*, 2009, vol. 113, 346-3 58
- **M. NAKAYAMA**. *Phys. Chem. Chem. Phys.*, 2012, vol. 14, 6079-6084
- **R. PLUGARU et al.** *Results Phys.*, 2012, vol. 2, 190-197
- **G. PAASCH et al.** *Synth Met.*, 2002, vol. 132, 97-104